# EUROPEAN PATENT SPECIFICATION

(11) **EP 3 171 854 B1**
(45) Date of publication and mention of the grant of the patent: **04.03.2020**
(21) Application number: 15723639.9
(22) Date of filing: 08.05.2015
(51) Int. Cl.: A61K 8/365, A61K 8/43, A61K 8/67, A61Q 11/00, A61K 8/73, A61K 8/81, A61K 8/23, A61K 31/155

(54) **IMPROVED MOUTHWASH PREPARATION**
VERBESSERTES MUNDWASSERPRÄPARAT
PRÉPARATION POUR BAIN DE BOUCHE AMÉLIORÉE

(43) Date of publication of application: 31.05.2017
(73) Proprietor: Curasept Ads S.r.L., 20147 Saronno Varese (IT)
(72) Inventor: BOIOCCHI, Lorenzo, I-20147 Saronno Varese (IT)
(74) Representative: Capasso, Olga
(86) International application number: PCT/EP2015/060260
(87) International publication number: WO 2016/112998

(56) References cited:
- EP-A1- 2 614 812
- WO-A2-2009/106963

## Description

### Background of the art

Chlorhexidine is known since more than twenty years as the gold standard for its antiplaque and antibacterial activity.

It is in fact capable of inhibiting the formation of new plaque and of disaggregating the layers already deposited on the tooth surface acting on its matrix thanks to its competitive mechanism with the calcium ions.

Chlorhexidine based mouthwashes are very useful in the treatment of periodontal pathologies and for enhancing the post-surgical healing process.

The antiseptic effect of chlorhexidine is due to its ability to establish chemical bonds with anionic groups (phosphate, sulfate, carboxyl group) present at the level of the bacterial cell wall and to induce a marked increase in cell permeability and alteration of the osmotic equilibrium.

Chlorhexidine has however the major drawback of causing brownish staining on the surface of the teeth as well as on prosthesis and tongue, which reduce significantly the patient compliance.

In order to overcome said problem, anti-discolorant systems (ADS) have been developed, which comprise adding in the chlorhexidine formulation sodium metabisulphite, for its property of preventing the Maillard reaction, and ascorbic acid, which interfere with the formation of ferric disulfur organic compound.

EP 2 614 812 discloses a mouthwash against plaque formation and pigmentation comprising chlorhexidine or a salt or a complex thereof, an alkaline or alkaline earth metal metabisulfite salt, ascorbic acid and hyaluronic acid or a salt or complex thereof.

Though the above, there is the need of improved mouthwash preparations, which could solve the problem of the teeth discoloration.

### Summary of the invention

It has been surprising found that a combination of a metabisulfite salt of an alkali or an alkaline-earth metal thereof, ascorbic acid or an alkali or an alkaline-earth metal salt thereof, hyaluronic acid or a salt thereof and polyvinylpirrolidone-vinylacetate can be used as an improved anti-pigmentation system in a mouthwash preparation based on chlorhexidine.

### Brief description of the figures

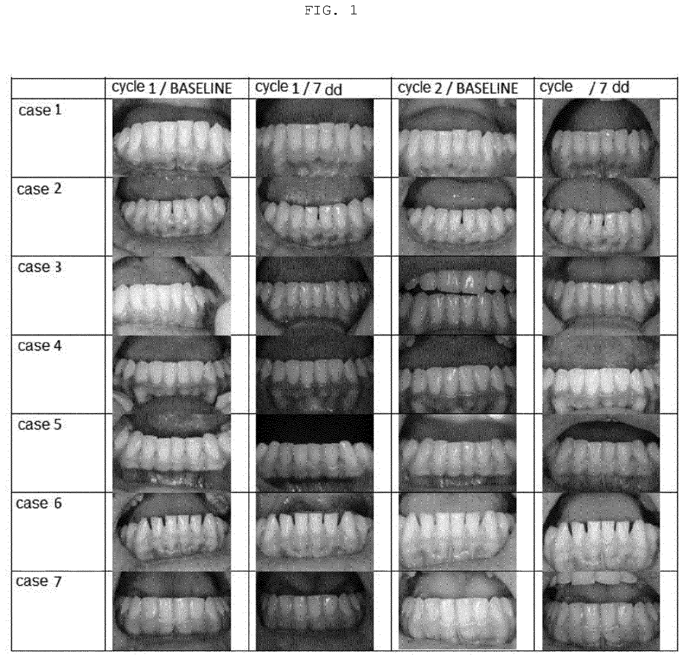

Fig. 1 shows the results of the test on the composition of the invention.

### Object of the invention

It is herein disclosed an anti-pigmentation system for a chlorhexidine based mouthwash.

The mouthwash comprising said system represents itself an object of the invention.

It is also disclosed a process for the preparation of a mouthwash based on chlorhexidine and comprising the anti-pigmentation system herein disclosed.

It is also disclosed a method for the prevention and treatment of plaque comprising the use of the mouthwash of the invention.

It is also disclosed a method for the cosmetic prevention and treatment of plaque comprising the use of the mouthwash of the invention.

It is also disclosed a method for the prevention of stains on the surface of the teeth comprising the use of the mouthwash of the invention.

It is also disclosed a method for the cosmetic prevention of stains on the surface of the teeth comprising the use of the mouthwash of the invention.

In addition, it is disclosed the use of the mouthwash of the invention for the protection of gingiva and oral mucosa and for the prevention and treatment of gingivitis.

### Detailed description of the invention

It is disclosed an anti-pigmentation system that can be used for preparing chlorhexidine based mouthwashes.

In particular, according to the present invention, the mouthwash comprises an amount of chlorhexidine of from about 0.01% to 0.3% (w/w) and preferably of from about 0.09% to 0.25% (w/w).

According to an even preferred embodiment, chlorhexidine is comprised in a quantity of about 0.2% (w/w) .

Chlorhexidine may be present as such or in the form of a salt or a complex thereof.

A salt may be represented by chlorhexidine digluconate.

As per the anti-pigmentation system herein disclosed, it is represented by a composition comprising: a metabisulfite salt of an alkali or an alkaline-earth metal, ascorbic acid or an alkali or an alkaline-earth metal salt thereof, hyaluronic acid or a salt thereof and polyvinylpirrolidone-vinylacetate.

In particular, said metabisulfite salt can be present in a quantity of about 0.1% to about 0.5% and preferably of about 0.15% to about 0.28% (w/total volume of the mouthwash).

In a preferred embodiment, the metabisulfite salt is represented by sodium metabisulfite.

In a particular embodiment, the ascorbic acid or the salt above referred can be substituted, partially or completely, by citric acid and/or an alkali or an alkaline-earth metal salt thereof.

For instance, sodium citrate tribasic can be used.

In a preferred embodiment, the ascorbic acid and a salt thereof, can be present in a quantity of about 1.0% to about 1.5% and preferably of about 1.0% to about 1.2% (total weight of the acid plus salt/total volume of the mouthwash).

In the other preferred embodiment, citric acid can be present in a quantity of about 0.01% to about 0.02% (w/total volume of the mouthwash), while if present, a citrate salt can be present in a quantity of about 0.8% to about 2.0% and preferably of about 1% to about 1.5% (w/total volume of the mouthwash).

As per the hyaluronic acid, it can be present in the form of sodium or potassium salt or a mixture of the two salts.

Preferably, the hyaluronic acid or the hyaluronate salt is present in an amount of about 0.01% to about 1.5% and preferably of about 0.05% to about 1.2% (w/total volume of the mouthwash).

The mouthwash of the invention may further comprise additives like: sweeteners and/or flavorings, a source of fluorine, humectant, preservatives, surfactants, pH regulators, coloring agents.

Preferably, flavorings are present in an amount of about 0.05-0.15% (w/total volume of the mouthwash) and can be selected in the group comprising peppermint, menthol, anethole, *viridis* mint, cinnamon, clove, eucalyptus, etc.

Sweeteners can be present in a quantity of about 6-10% (w/total volume of the mouthwash).

Sweeteners can be selected in the group comprising non-cariogenic sugars like xylitol, saccharin or an alkali or alkaline-earth metal salts thereof, acesulfame or an alkali or alkaline-earth metal salts thereof, sucralose, Stevia rebaudiana extract.

The source of fluorine can be represented by fluoride or alkali or alkaline-earth metal salts thereof.

Humectants can be present in a quantity of about 0.1-0.2% (w/total volume of the mouthwash).

Preferably, the humectant may be selected in the group comprising sorbitol, glycerin or propylene glycol.

Preservatives may be selected in the group comprising the benzoates, phenoxyethanol, chlorphenesin.

The surfactants are present in an amount of about 1.0-3% (w/total volume of the mouthwash).

The surfactants may be selected in the group comprising hydrogenated castor oil PEG40, Poloxamer 407 or other surfactants suitable for the purposes of the present invention.

pH regulators can be selected in the group comprising citric acid and its salts, like sodium salt.

Coloring agents may be present in an amount of about 0.0005-0.001% (w/total volume of the mouthwash) selected among those agents suitable for use in the field, like for instance CI 1940, CIU 42090, CI 17200.

As per the polyvinylpirrolidone-vinylacetate, according to the present invention, it is comprised in an amount of about 0.02% to about 0.5% (w/total weight of the mouthwash).

Preferably, the polyvinylpirrolidone-vinylacetate may be comprised in an amount of about 0.15% to about 0.2% and more preferably it is comprised in an amount of about 0.09% to about 0.12% (w/total weight of the mouthwash).

As above indicated, the mouthwash comprising the anti-pigmentation system disclosed represents an object of the invention.

It is also disclosed a process for the preparation of a mouthwash based on chlorhexidine and comprising the anti-pigmentation system herein disclosed.

It is also disclosed a method for the prevention and treatment of plaque comprising the use of the mouthwash of the invention.

It is also disclosed a method for the cosmetic prevention and treatment of plaque comprising the use of the mouthwash of the invention.

It is also disclosed a method for the prevention of stains on the surface of the teeth comprising the use of the mouthwash of the invention.

It is also disclosed a method for the cosmetic treatment of stains on the surface of the teeth comprising the use of the mouthwash of the invention.

It is also disclosed the use of the mouthwash according to the invention for the therapeutic prevention and treatment of plaque.

It is also disclosed the use of the mouthwash of the invention for the cosmetic prevention and treatment of plaque.

It is also disclosed the use of the mouthwash of the invention for the prevention of stains on the surface of the teeth.

It is also disclosed the use of the mouthwash according to the invention for the cosmetic prevention of stains on the surface of the teeth.

It is also disclosed the use of the mouthwash of the invention for the prevention, protection and treatment of gingivitis.

In particular, the use of the disclosed mouthwash once or twice a day for at least one minute and for a period of from 7 to 15 days may be helpful in the prevention and treatment of plaque and/or of gengivitis, in the prevention of the bacteria growth, as an antibacterial.

The mouthwash of the invention can be used in the treatment of parodontitis when used once or twice a day for at least one minute and a period of from 7 to 15 days at the concentration of 0.2% followed by the use once or twice a day for at least one minute for about 21 days at the concentration of 0.12%.

It is also disclosed the use of polyvinylpirrolidone-vinylacetate combined with chlorhexidine in a mouthwash for the treatment of or the prevention of the formation of plaque.

### EXAMPLE 1

According to the above description, the following mouthwash preparation can be:

| **COMPONENT** | **QUANTITY** |
|---|---|
| water | q.b. to 100% |
| sugar | 6-10% |
| humectants | 0.1-0.2% |
| surfactants | 1.0-3% |
| Ascorbic acid | 0.1-1.5% (w/v) |
| Chlorhexidine digluconate | 0.05-0.3% (w/w) |
| PVP-VA copolymer | 0.05-0.3% |
| sodium hyaluronate | 0.05-1.2% |
| arome | 0.05-0.15% |
| sodium metabisulfite | 0.1-0.5% (w/v) |
| sodium citrate | 1.0-1.5% |
| citric acid | 0.01-0.015 |
| coloring agent | 0.0005-0.0009% |

### EXAMPLE 2

The purpose of the clinical trial was to evaluate the effectiveness against pigmentations by a dental mouthwash containing 0.2% chlorhexidine, PVP-VA, hyaluronic acid and ADS® (Anti Discoloration System) compared to a mouthwash only containing 0.2% chlorhexidine and the ADS® system.

### Materials and Methods

17 volunteers have been enrolled in the study, selected at the Clinica Universitaria at the IRCCS Istituto Ortopedico Galeazzi (Milan).

Patients had to meet the following requirements:
1. Patients in good health, absence of systemic diseases;
2. Adhesion through informed consent;
3. Older than 18 years;
4. Non-smokers or light smokers (less than 5 cigarettes a day);
5. Good oral hygiene with low levels of plaque index (FMPS FMBS and ≤ 25%).

Before the study, patients have been subjected to professional oral hygiene session, in order to remove possibly plaque and pigmentations already present on the dental surface. There have been prepared anonymous packs marked only with two letters:

| | |
|---|---|
| **A**: | mouthwash (HA + ADS) |
| **B**: | mouthwash (ADS) |

Patients volunteers have been then divided into two groups randomly and they have been given a box corresponding to the randomization list.

Patients have been asked to suspend the operations of mechanical oral hygiene in the V sextant, i.e. only in the lower 33 to 43 (6 teeth in all) .

In the remaining areas, the subjects continued to brush but with no toothpaste.

Everyone handed an identical toothbrush: model Curaprox 5460.

Patients have thus made a first cycle of rinses keeping the product in the mouth for one minute, twice a day, for 7 days, after which they recurred to the center investigator for clinical evaluations.

Soon after, a second session of oral hygiene was performed, to remove the stains and plaque formed, and the patient have been asked to recur after another 7 days.

So, with the same mode of the previous rinse cycle, the patient have made a second rinse cycle with the mouthwash that had not received during the first cycle.

During the visits at time zero (baseline) and after 7 days there have been detected the plaque index (PI, according to Loe and Silness, 1964), gingival index (GI, according to Loe, 1967) and the index of pigmentation (SI, according to Lobene, 1968).

Information has been gathered about any adverse events or problems that emerged during the study through a questionnaire.

In this, patients have noted also the intake of chromogenic substances such as coffee, tea, cigarettes, red wine, spinach and green vegetables in general.

At each session, intraoral photographs have been taken for the assessment of the pigmentation and the same has been made for the evaluation of the extent of pigmentation. The assessment of the Stain Index has been independently performed by 3 different experts operators.

### Results

Of the 17 subjects enrolled, 4 failed to meet the protocol because they performed mechanical procedures for the oral hygiene on the affected area, and have been therefore excluded. The remaining 13 subjects in the study completed the rinse cycles with good compliance.

The results are shown in Figure 1.

With respect to the accumulation of plaque, both mouthwashes proved to be effective in combating the dental plaque, which resulted mostly present at 7 days, although there was no evidence of any substantial deposits for the both 2 mouthwashes.

Even the Gingival Index was similar in the 2 mouthwashes, but in this case the presence of inflammation has been detected only in one patient in the group A and two patients in group B.

Regarding the Stain Index, a trend towards less pigmentation has been detected for the mouthwash with ADS + Hyaluronic Acid + PVP-VA. The level of pigmentation tends to be low also for the mouthwash with only ADS, the effectiveness of which has been known for a long time.

| | PI 7 days | GI 7 days | SI 7 days |
|---|---|---|---|
| Mouthwash A | 1.3 | 0.3 | 0.2 |
| Mouthwash B | 1.4 | 0.2 | 0.4 |

The values of PI, GI and SI have been determined considering a modification of at least 30% of the dental elements in order to give a value corresponding to an index in the corresponding scale (values 1, 2 or 3). For instance, in a healthy subject with 28 dental elements (teeth) at least 10 teeth must have slight stain in order to be given value 1 of Stain Index, to the contrary, 0 is given.

According to the above, there will be evident to the person skilled in the art the advantages provided by the anti-pigmentation system herein disclosed.

First of all, the mouthwash comprising said system has proved to limit to a much wider extent the formation of stain on the tooth surface, with respect to a mouthwash comprising an anti-pigmentation system that does not include hyaluronic acid.

When polyvinylpirrolidone-vinylacetate is also included in the anti-pigmentation system herein disclosed, it has been observed a synergic action with chlorhexidine against bacteria and against the formation of the bacterial biofilm.

In addition to that, polyvinylpirrolidone-vinylacetate has also been seen as acting in synergy with hyaluronic acid in the formation of a mechanical barrier, which can protect both the teeth surface and the soft tissues (gingiva and oral mucosa).

Polyvinylpirrolidone-vinylacetate (PVP-VA) is a safe ingredient: no adverse reactions after intake are known at present, PVP VA is widely used on industrial scale in cosmetics, food preparations and medications. The value of the acceptable daily dose is high (i.e., 50 mg/kg), and is far above the values established for food and pharmacological products. In addition, the component is highly soluble in water.

Surprisingly, the above advantageous effects have been noticed to be not to the detriment of the antibacterial effect.

## Claims

1. A mouthwash comprising chlorhexidine in a quantity of about 0.01% to 0.3%(w/w), preferably of about 0.09% to about 0.25% (w/w), and an anti-pigmentation system, said anti-pigmentation system comprising a metabisulfite salt of an alkali or an alkaline-earth metal, ascorbic acid or an alkali or an alkaline-earth metal salt thereof, hyaluronic acid or a salt thereof and polyvinylpirrolidone-vinylacetate, wherein polyvinylpirrolidone-vinyl acetate is comprised in an amount of about 0.02% to about 0.5% (w/total weight of the mouthwash), preferably in an amount of about 0.15% to about 0.2% and more preferably of about 0.09% to about 0.12% (w/total volume of the mouthwash) .

2. The mouthwash according to the preceding claim, wherein chlorhexidine is in the form of a salt or a complex thereof.

3. The mouthwash according to the preceding claim 1 or 2, wherein chlorhexidine is in the form of chlorhexidine digluconate.

4. The mouthwash according to any one of the preceding claims 1 to 3, wherein the metabisulfite salt is comprised in an amount of about 0.1% to about 0.5% and preferably of about 0.15% to about 0.28% (w/total volume of the mouthwash) .

5. The mouthwash according to the preceding claims 1 to 4, wherein the metabisulfite salt is sodium metabisulfite.

6. The mouthwash according to any one of the preceding claims 1 to 5, wherein the ascorbic acid or salt thereof can be present in a quantity of about 1.0% to about 1.5% and preferably in an amount of about 1.0% to about 1.2% (total weight acid and salt/total volume of the mouthwash).

7. The mouthwash according to the preceding claim, wherein the ascorbic acid or a salt thereof, can be substituted, partially or completely, by citric acid or an alkali or an alkaline-earth metal salt thereof.

8. The mouthwash according to the preceding claim 7, wherein the alkali or the alkaline- earth metal salt of citric acid is sodium citrate tribasic.

9. The mouthwash according to the preceding claims 7 or 8, wherein the citric acid can be present in a quantity of about 0.01% to about 0.02% (weight/total volume of the mouthwash), while if present a citrate salt can be present in a quantity of about 0.8% to about 2.0% and preferably of about 1% to about 1.5% (w/total volume of the mouthwash).

10. The mouthwash according to any one of the preceding claims 1 to 9, wherein the hyaluronic acid salt can be present in the form of sodium or potassium salt or a mixture of the two salts.

11. The mouthwash according to any one of the preceding claims 1 to 10, wherein hyaluronic acid or a salt thereof is comprised in an amount of about 0.01% to about 1.5% and preferably of about 0.05% to about 1.2% (w/total volume of the mouthwash) .

12. The mouthwash according to any one of the preceding claims 1 to 11, further comprising one or more of the following additives: sweeteners and/or flavorings, a source of fluorine, humectants, preservatives, surfactants, pH regulators, coloring agents.

13. The mouthwash according to the preceding claim, wherein the source of fluorine is represented by fluoride or alkali or alkaline-earth metal salts thereof.

14. The mouthwash as defined in any one of the preceding claims 1 to 13 for use in the prevention and treatment of plaque.

15. The mouthwash according to any one of the preceding claims 1 to 13 for use in the prevention of stains on the surface of the teeth or in the prevention and treatment of gingivitis.

## Patentansprüche

1. Mundwasser umfassend Chlorhexidin in einer Menge von etwa 0,01 % bis 0,3 % (Gew./Gew.), bevorzugt von etwa 0,09 % bis etwa 0,25 % (Gew./Gew.) und ein Anti-Pigmentiersystem, wobei das Anti-Pigmentiersystem ein Metabisulfitsalz eines Alkali- oder eines Erdalkalimetalls, Ascorbinsäure oder ein Alkali- oder ein Erdalkalimetallsalz davon, Hyaluronsäure oder ein Salz davon und Polyvinylpyrrolidon-Vinylacetat umfasst, wobei Polyvinylpyrrolidon-Vinylacetat in einer Menge von etwa 0,02 % bis etwa 0,5 % (Gew./Gesamtgewicht des Mundwassers), bevorzugt in einer Menge von etwa 0,15 % bis etwa 0,2 % und noch bevorzugter etwa 0,09 % bis etwa 0,12 % (Gew./Gesamtvolumen des Mundwassers) enthalten ist.

2. Mundwasser nach dem vorhergehenden Anspruch, wobei Chlorhexidin in Form eines Salzes oder eines Komplexes davon vorliegt.

3. Mundwasser nach dem vorhergehenden Anspruch 1 oder 2, wobei Chlorhexidin in Form von Chlorhexidindigluconat vorliegt.

4. Mundwasser nach irgendeinem der vorhergehenden Ansprüche 1 bis 3, wobei das Metabisufitsalz in einer Menge von etwa 0,1 % bis etwa 0,5 % und bevorzugt von etwa 0,15 % bis etwa 0,28 % (Gew./Gesamtvolumen des Mundwassers) enthalten ist.

5. Mundwasser nach den vorhergehenden Ansprüchen 1 bis 4, wobei das Metabisufitsalz Natriummetabisufit ist.

6. Mundwasser nach irgendeinem der vorhergehenden Ansprüche 1 bis 5, wobei die Ascorbinsäure oder das Salz davon in einer Menge von etwa 1,0 % bis etwa 1,5 % und bevorzugt in einer Menge von etwa 1,0 % bis etwa 1,2 % (Gesamtgewicht Säure und Salz/Gesamtvolumen des Mundwassers) vorliegen kann.

7. Mundwasser nach dem vorhergehenden Anspruch, wobei die Ascorbinsäure oder ein Salz davon durch Zitronensäure oder ein Alkali- oder ein Erdalkalimetallsalz davon teilweise oder vollständig substituiert sein kann.

8. Mundwasser nach dem vorhergehenden Anspruch 7, wobei das Alkali- oder das Erdalkalimetallsalz von Zitronensäure dreibasisches Natriumcitrat ist.

9. Mundwasser nach den vorhergehenden Ansprüchen 7 oder 8, wobei die Zitronensäure in einer Menge von etwa 0,01 % bis etwa 0,02 % (Gewicht/Gesamtvolumen des Mundwassers) vorliegen kann, während, liegt es vor, ein Citratsalz in einer Menge von etwa 0,8 % bis etwa 2,0 % und bevorzugt von etwa 1 % bis etwa 1,5 % (Gew./Gesamtvolumen des Mundwassers) vorliegen kann.

10. Mundwasser nach irgendeinem der vorhergehenden Ansprüche 1 bis 9, wobei das Hyaluronsäuresalz in Form von Natrium- oder Kaliumsalz oder einer Mischung der beiden Salze vorliegen kann.

11. Mundwasser nach irgendeinem der vorhergehenden Ansprüche 1 bis 10, wobei Hyaluronsäure oder ein Salz davon in einer Menge von etwa 0,01 % bis etwa 1,5 % und bevorzugt etwa 0,05 % bis etwa 1,2 % (Gew./Gesamtvolumen des Mundwassers) enthalten ist.

12. Mundwasser nach irgendeinem der vorhergehenden Ansprüche 1 bis 11, ferner ein oder mehrere der folgenden Zusatzmittel umfassend: Süßungsmittel und/oder Geschmacksmittel, eine Quelle von Fluor, Feuchthaltemittel, Konservierungsmittel, Tenside, pH-Wertregulatoren, Farbmittel.

13. Mundwasser nach dem vorhergehenden Anspruch, wobei die Quelle von Fluor durch Fluorid oder Alkali- oder Erdalkalimetallsalze davon dargestellt ist.

14. Mundwasser wie in irgendeinem der vorhergehenden Ansprüche 1 bis 13 definiert, zur Verwendung zur Verhinderung und Behandlung von Zahnbelag.

15. Mundwasser nach irgendeinem der vorhergehenden Ansprüche 1 bis 13 zur Verwendung zur Verhinderung von Flecken auf der Oberfläche der Zähne oder zur Verhinderung und Behandlung von Zahnfleischentzündung.

## Revendications

1. Bain de bouche comprenant de la chlorhexidine en une quantité d'environ 0,01 % à 0,3 % (p/p), de préférence d'environ 0,09 % à environ 0,25 % (p/p), et un système anti-pigmentation, ledit système anti-pigmentation comprenant un sel de métabisulfite d'un métal alcalin ou alcalino-terreux, de l'acide ascorbique ou un sel de métal alcalin ou alcalino-terreux de celui-ci, de l'acide hyaluronique ou un sel de celui-ci et du polyvinylpyrrolidone-acétate de vinyle, dans lequel le polyvinylpyrrolidone-acétate de vinyle est compris en une quantité d'environ 0,02 % à environ 0,5 % (p/poids total du bain de bouche), de préférence en une quantité d'environ 0,15 % à environ 0,2 % et plus préférablement d'environ 0,09 % à environ 0,12 % (p/volume total du bain de bouche).

2. Bain de bouche selon la revendication précédente, dans lequel la chlorhexidine est sous la forme d'un sel ou d'un complexe de celle-ci.

3. Bain de bouche selon la revendication 1 ou 2 précédente, dans lequel la chlorhexidine est sous la forme de digluconate de chlorhexidine.

4. Bain de bouche selon l'une quelconque des revendications 1 à 3 précédentes, dans lequel le sel de métabisulfite est compris en une quantité d'environ 0,1 % à environ 0,5 % et de préférence d'environ 0,15 % à environ 0,28 % (p/volume total du bain de bouche).

5. Bain de bouche selon l'une quelconque des revendications 1 à 4 précédentes, dans lequel le sel de métabisulfite est le métabisulfite de sodium.

6. Bain de bouche selon l'une quelconque des revendications 1 à 5 précédentes, dans lequel l'acide ascorbique ou un sel de celui-ci peut être présent en une quantité d'environ 1,0 % à environ 1,5 % et de préférence en une quantité d'environ 1,0 % à environ 1,2 % (poids total de l'acide et du sel/volume total du bain de bouche).

7. Bain de bouche selon l'une quelconque des revendications précédentes, dans lequel l'acide ascorbique ou un sel de celui-ci peut être substitué, partiellement ou totalement, par de l'acide citrique ou un sel de métal alcalin ou alcalino-terreux de celui-ci.

8. Bain de bouche selon la revendication 7 précédente, dans lequel le sel de métal alcalin ou alcalino-terreux d'acide citrique est le citrate de sodium tribasique.

9. Bain de bouche selon la revendication 7 ou 8 précédente, dans lequel l'acide citrique peut être présent en une quantité d'environ 0,01 % à environ 0,02 % (poids/volume total du bain de bouche), tandis que s'il est présent un sel de citrate peut être présent en une quantité d'environ 0,8 % à environ 2,0 % et de préférence d'environ 1 % à environ 1,5 % (p/volume total du bain de bouche).

10. Bain de bouche selon l'une quelconque des revendications 1 à 9 précédentes, dans lequel le sel de l'acide hyaluronique de celui-ci peut être présent sous la forme d'un sel de sodium ou de potassium ou d'un mélange des deux sels.

11. Bain de bouche selon l'une quelconque des revendications 1 à 10 précédentes, dans lequel l'acide hyaluronique ou un sel de celui-ci est compris en une quantité d'environ 0,01 % à environ 1,5 % et de préférence d'environ 0,05 % à environ 1,2 % (p/volume total du bain de bouche).

12. Bain de bouche selon l'une quelconque des revendications 1 à 11 précédentes, comprenant en outre un ou plusieurs des additifs suivants : les édulcorants et/ou les agents aromatisants, une source de fluor, les agents humectants, les conservateurs, les tensioactifs, les régulateurs de pH, les agents colorants.

13. Bain de bouche selon la revendication précédente, dans lequel la source de fluor est représentée par le fluorure ou des sels de métal alcalin ou alcalino-terreux de celui-ci.

14. Bain de bouche selon l'une quelconque des revendications 1 à 13 précédentes pour une utilisation dans la prévention et le traitement de la plaque dentaire.

15. Bain de bouche selon l'une quelconque des revendications 1 à 13 précédentes pour une utilisation dans la prévention et le traitement des taches sur la surface des dents ou dans la prévention et le traitement d'une gingivite.
